# EUROPÄISCHE PATENTSCHRIFT

(11) **EP 2 113 265 B1**
(45) Veröffentlichungstag und Bekanntmachung des Hinweises auf die Patenterteilung: **02.11.2011**
(21) Anmeldenummer: 09004828.1
(22) Anmeldetag: 01.04.2009
(51) Int. Cl.: A61M 1/00, A61M 39/02, A61M 39/12

(54) **Handgriff für ein medizinisches Instrument**
Handle for a medical instrument
Poignée pour un instrument médical

(30) Priorität: 29.04.2008 DE 202008005899 U
(43) Veröffentlichungstag der Anmeldung: 04.11.2009
(73) Patentinhaber: Karl Storz GmbH & Co. KG, 78532 Tuttlingen (DE)
(72) Erfinder: Blocher, Martin, 78532 Tuttlingen (DE); Volkmer, Dominik, 78567 Fridingen a.D. (DE); Bacher, Uwe, 78532 Tuttlingen (DE)
(74) Vertreter: Hofmeister, Frank

(56) Entgegenhaltungen:
- WO-A1-2009/000439
- DE-A1- 19 647 816
- US-A- 4 723 948

## Beschreibung

Die Erfindung betrifft einen Handgriff für ein medizinisches Instrument mit einem über einen Deckel verschließbaren Griffgehäuse und mindestens einem im Griffgehäuse an einen Schlauchanschlussstutzen anschließbaren Saug- und/oder Spülschlauch, wobei jeder Saug- und/oder Spülschlauch über einen Adapter an den zugehörigen Schlauchanschlussstutzen anschließbar ist.

Um insbesondere bei endoskopischen Operationen dem Operateur einen guten Blick auf das Operationsgebiet zu gewähren, ist es häufig erforderlich, das Operationsgebiet mit einer Spülflüssigkeit zu reinigen und die Spülflüssigkeit sowie austretendes Blut aus dem Operationsgebiet abzusaugen. Zu diesem Zweck sind entsprechende medizinische Instrumente mit Saug- und/oder Spülschläuchen ausgestattet, die über die Handhabe des jeweiligen Instruments bedienbar sind.

Bei den aus der Praxis bekannten medizinischen Instrumenten besteht das Problem, dass diese im Bereich des die Schlauchanschlussstutzen aufweisenden Handgriffs schlecht zu reinigen ist und darüber hinaus eine nur unzureichende Fluiddichtigkeit gegen Leckagen gewährleisten.

Ein gattungsgemäßer Handgriff ist beispielsweise aus der DE 196 47 816 C2 bekannt. Bei diesem bekannten medizinischen Instrument sind im Inneren der Handhabe sind zwei Anschlussstutzen festlegbare Schläuche angeordnet. Am verschwenkbaren Gehäuseteil der Handhabe sind halbkreisförmige Druckflächen mit teilringförmigen Wulsten angeformt die im geschlossenen Zustand der Handhabe die Schläuche teilweise so umgreifen, dass das Schlauchmaterial radial gegen die Anschlussstutzen gepresst wird. Durch das Einwirken Druckflächen auf das Schlauchmaterial wird dieses im Bereich der Schlauchanschlussstutzten gestaucht.

Davon ausgehend liegt der Erfindung die **Aufgabe** zugrunde, einen Handgriff der eingangs genannten Art zu schaffen, der bei einfachem Aufbau eine zuverlässige fluiddichte Verbindung von Schlauch und Schlauchanschlussstutzen bei gleichzeitiger Schonung des Schlauchmaterials gewährleistet.

Die **Lösung** dieser Aufgabenstellung ist erfindungsgemäß **dadurch gekennzeichnet, dass** der zum Anschließen von zwei Schläuchen an zwei Schlauchanschlussstutzen ausgebildete Adapter beim Schließen des Griffgehäuses über einen am Deckel des Griffgehäuses angeordneten und direkt gegen den Adapter anlaufenden Anschlag gegen die Schlauchanschlussstutzen anpressbar ist, wobei der Adapter H-förmig oder U-förmig ausgebildet ist.

Durch die erfindungsgemäße Verwendung eines Adapters zum gleichzeitigen Anschließen von zwei Schläuchen an zwei zugehörige Schlauchanschlussstutzen werden die Schlauchenden geschont, da das eigentliche Kontaktieren mit den Schlauchanschlussstutzen über den zwischengeschalteten Adapter erfolgt. Durch die Ausbildung eines Anschlags am Deckel und/oder am Griffgehäuse, der beim Schließen des Griffgehäuses gegen den Adapter anläuft wird sichergestellt, dass automatisch bei jedem Schließen des Griffgehäuses der Adapter über dem Anschtag abdichtend und mit stets gleichem Druck gegen den zugehörigen Schlauchanschlussstutzen angepresst wird.

Gemäß einer bevorzugten Ausführungsform der Erfindung wird vorgeschlagen, dass der Anschlag als am Deckel angeformter, nach innen weisender Steg ausgebildet ist. Das Anordnen des Anschlags am Gehäusedeckel stellt eine besonders einfach zu fertigende und problemlos zu handhabende Ausgestaltungsform dar.

Alternativ zu der Anordnung des Anschlags am Deckel des Handgriffs ist es selbstverständlich auch möglich, den Anschlag am unteren Teil des Griffgehäuses so anzuordnen, dass der Anschlag beim Schließen des Deckels gegen den Adapter gedrückt wird.

Schließlich wird mit der Erfindung vorgeschlagen, dass am Adapter und/oder am Schlauchanschlussstutzen mindestens ein Dichtungselement, insbesondere eine O-Ring-Dichtung, festlegbar ist, um die fluiddichte Anlage des Adapters am zugehörigen Schlauchanschlussstutzen zu verbessern. Beim Anpressen des Adapters gegen den Schlauchanschlussstutzen wird das Dichtungselement abdichtend verformt.

Weiterhin wird mit der Erfindung vorgeschlagen, dass zum Abdichten gegenüber dem Schlauchanschlussstutzen der Adapter aus einem Dichtungsmaterial, insbesondere Silikon, besteht.

Weitere Merkmale und Vorteile der Erfindung ergeben sich anhand der zugehörigen Zeichnung, in der ein Ausführungsbeispiel eines erfindungsgemäßen Handgriffs für ein medizinisches Instrument nur beispielhaft dargestellt ist, ohne die Erfindung auf dieses Ausführungsbeispiel zu beschränken. In der Zeichnung zeigt:
- Fig. 1: eine teilweise geschnittene Seitenansicht eines erfindungsgemäßen Handgriffs mit geöffnetem Deckel;
- Fig. 2: eine Draufsicht auf den Handgriff gemäß Fig. 1; jedoch ohne Deckel;
- Fig. 3: eine Seitenansicht gemäß Fig. 1 mit teilweise geschlossenem Deckel;
- Fig. 4: eine Seitenansicht gemäß Fig. 3 mit geschlossenem Deckel;
- Fig. 5: eine vergrößerte Ansicht des Details V gemäß Fig. 4 und

- Fig. 6: eine vergrößerte schematische Ansicht einer zweiten Ausführungsform eines Adapters.

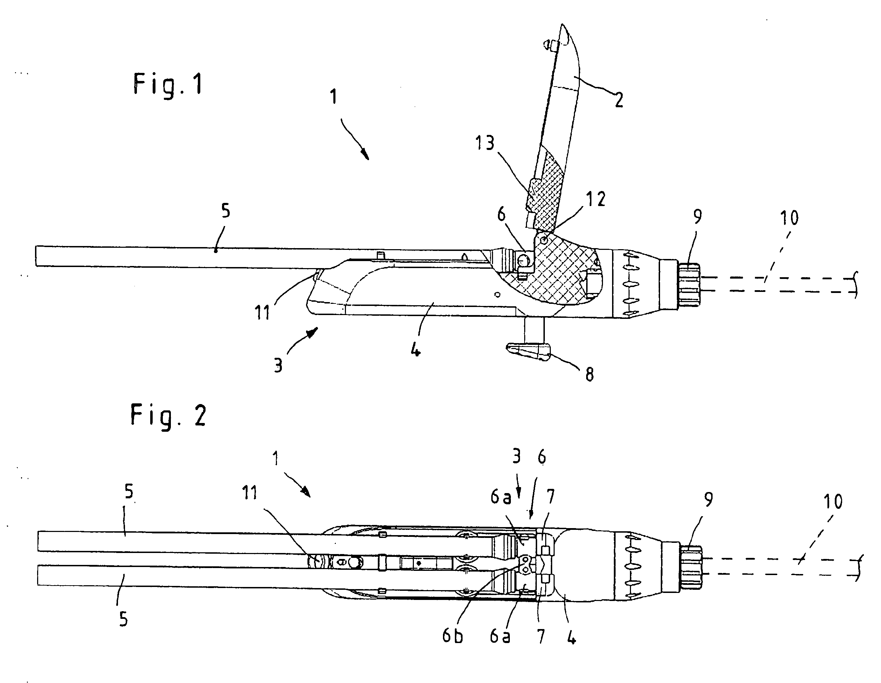

Die Abbildungen Fig. 1 bis Fig. 4 zeigen einen Handgriff 1 für ein medizinisches Instrument mit einem über einen Deckel 2 verschließbaren Griffgehäuse 3, wobei das Griffgehäuse 3 im Wesentlichen aus einem Basisteil 4 und dem Deckel 2 besteht.

Bei der dargestellten Ausführungsform des Handgriffs 1 sind im Griffgehäuse 3 zwei Saug- und Spülschläuche 5 über einen Adapter 6 an zwei Schlauchanschlussstutzen 7 anschließbar, jedoch sind auch Handgriffe 1 mit mehr als zwei Schläuchen 5 ausführbar. In jedem Fall ist aber das distalseitige Ende eines jeden Saug- und/oder Spülschlauches 5 an einem Adapter 6 festgelegt, der seinerseits an die zugehörigen Schlauchanschlussstutzen 7 anschließbar ist.

Die Verwendung des Adapters 6 zum Festlegen eines Saug- und Spülschlauches 5 am zugehörigen Schlauchanschlussstutzen 7 hat den Vorteil, dass das Anschließen des Saug- und Spülschlauches 5 am Schlauchanschlussstutzen 7 indirekt, nämlich über den zwischengeschalteten Adapter 6 erfolgt, so dass das Schlauchmaterial beim Anschließen geschont werden kann und es nicht einer jeweiligen Dehnung und Stauchung des Schlauchmaterials beim Aufschieben und Andrücken auf die Schlauchanschlussstutzen 7 bedarf.

Über die Saug- und Spülschläuche 5 wird Spülflüssigkeit zum Operationsgebiet geleitet und wieder aus dem Operationsgebiet abgesaugt, um dem Operateur eine gute Sicht auf das Operationsgebiet zu gewährleisten.

Zur Dosierung der über die Schläuche 5 zu bewerkstelligenden Saug- und/oder Spülleistung weist der Handgriff 1 weiterhin zwei am Basisteil 4 angeordnete Taster 8 auf, über die der Durchfluss durch die Schläuche 5 drosselbar ist. In den Darstellungen gemäß Fig. 1, 2 und 4 ist jeweils nur ein Taster 8 zu erkenne, da die Taster in diesen Seitenansichten direkt hintereinander angeordnet sind. Die Anzahl der Taster 8 entspricht aber in der Regel der Anzahl der im Handgriff 1 angeordneten Schläuche 5.

Am distalen Ende weist der Handgriff 1 eine Kupplungselement 9 in Form einer Klemmschraube auf, über das beispielsweise ein Instrumentenschaft 10 (gestrichelt dargestellt) am Handgriff 1 festlegbar ist.

Wie aus Fig. 2 ersichtlich, ist der Adapter 6 bei der hier dargestellten Ausführungsform zur Kopplung von zwei Schläuchen 5 mit zwei Schlauchanschlussstutzen 7 H-förmig so ausgebildet, dass die Schläuche 5 auf die proximalseitigen freien Enden der Längsschenkel 6a des H-förmigen Adapters 6 aufgeschoben werden, wohingegen die distalseitigen freien Enden der Längsschenkel 6a des H-förmigen Adapters 6 zur Kopplung mit den beiden Schlauchanschlussstutzen 7 dienen.

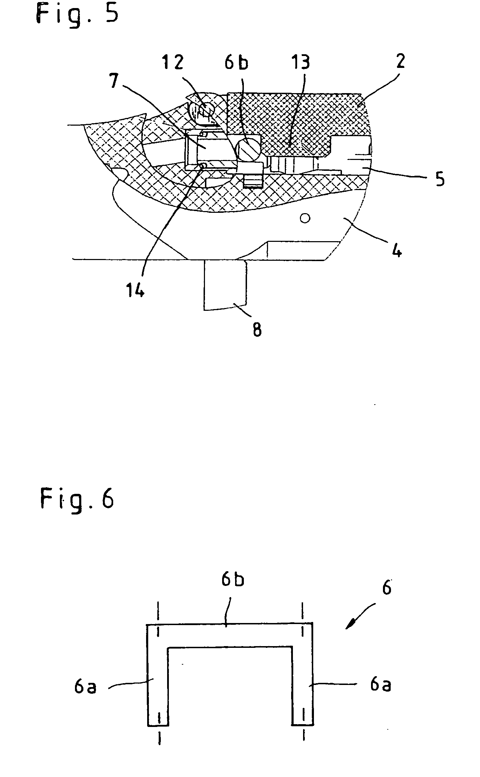

Bei der in Fig. 6 dargestellten alternativen Ausführungsform ist der Adapter 6 U-förmig so ausgebildet, dass die Schläuche 5 auf die proximalseitigen freien Enden der Längsschenkel 6a des U-förmigen Adapters 6 aufgeschoben werden, wohingegen distalseitig ein die beiden Längsschenkel 6a des U-förmigen Adapters 6 miteinander verbindender Verbindungssteg 6b zur Kopplung mit den beiden Schlauchanschlussstutzen 7 dient.

Das Anschließen der Schläuche 5 an die Schlauchanschlussstutzen 7 des Handgriffs 1 geschieht wie folgt:
Im ersten Arbeitsschritt wird ein Sperrmechanismus 11 am Griffgehäuse 3 betätigt, um den Deckel 2 aus seiner Verrastung am Basisteil 4 zu entriegeln. Danach lässt sich der Deckel 2 um eine Schwenkachse 12 um etwa 90° gegenüber dem Basisteil 4 in die geöffnete Position verschwenken, wie sie in Fig. 1 dargestellt ist.
Nachfolgend werden die beiden Schläuche 5 von derselben Seite her so weit wie möglich auf die freien Enden der Längsschenkel 6a des H- oder U-förmigen Adapters 6 aufgeschoben. Dieses solchermaßen mit dem Adapter 6 verbundene Schlauchset wird jetzt so in das Basisteil 4 des Handgriffs 1 eingelegt, dass die nicht mit den Schläuchen 5 bestückten freien Enden der Längsschenkel 6a des H-förmigen Adapters 6 an den Schlauchanschlussstutzen 7 anliegen, wie dies der Draufsicht gemäß Fig. 2 zu entnehmen ist. Um die Schläuche 5 entsprechend ihrer Funktion als Saug- oder Spülschlauch 5 an den richtigen Schlauchanschlussstutzen 7 anzuschließen, weist mindestens ein Schlauch 5 eine Farb- oder Zeichencodierung auf, die sich entsprechend am zugehörigen Schlauchanschlussstutzen 7 wiederfindet.

Entscheidend für den fluiddichten Anschluss des Adapters 6 an die Schlauchanschlussstutzen 7 ist, dass der Adapter 6 ausreichend tief in die Schlauchanschlussstutzen 7 eingeschoben wird.

Zu diesem Zweck ist auf der dem Innenraum des Griffgehäuses 3 zugewandten Innenseite des Deckels 2 ein Anschlag 13 angeformt, der bei der dargestellten und aus Fig. 1, 3, 4 und 5 erkennbaren Ausführungsform als schmaler Steg ausgebildet ist.

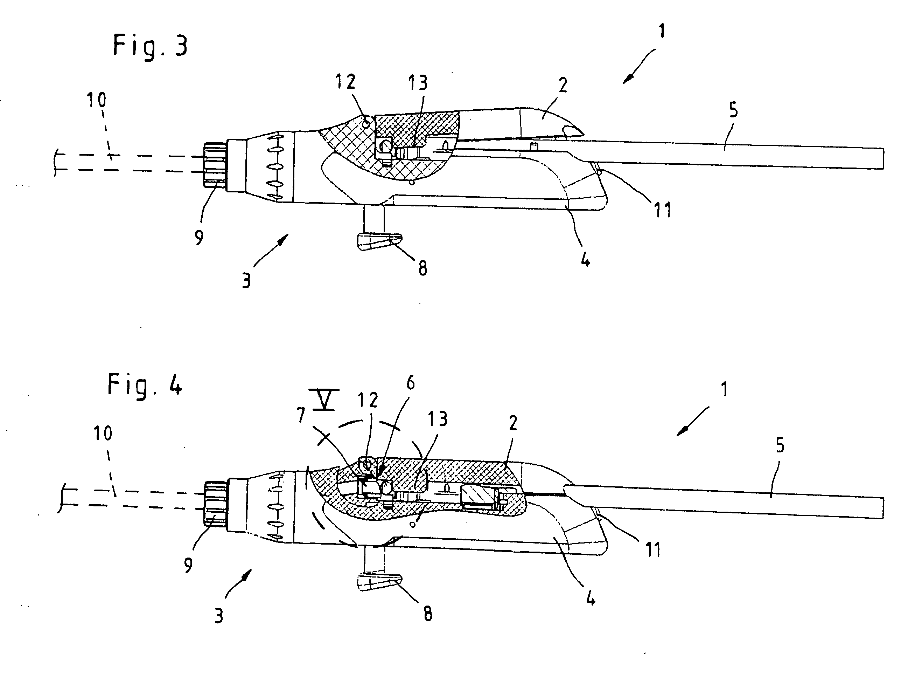

Dieser Anschlag 13 ist so an der Innenseite des Deckels 2 positioniert, dass er beim Schließen des Deckels, wie dies in Fig. 3, 4 und 5 dargestellt ist, gegen den die beiden Längsschenkel 6a des H- oder U-förmigen Adapters 6 miteinander verbindenden Verbindungssteg 6b anläuft und so den Adapter 6 nach distal gegen die Schlauchanschlussstutzen 7 presst.

Der Anschlag 13 bewirkt somit, dass automatisch beim Schließen des Deckels 2 der mit den Schläuchen 5 bestückte Adapter 6 in fluiddichte Anlage gegen die Schlauchanschlussstutzen 7 gepresst wird. Wie aus Fig. 4 und 5 ersichtlich, liegt der Anschlag 13 bei geschlossenem Deckel 2 am Verbindungssteg 6b des Adapters 6 an und stellt somit sicher, dass der Adapter 6 auch durch Zugbelastung in proximale Richtung an den Schläuchen 5 nicht aus seiner fluiddichten Anlage an den Schlauchanschlussstutzen 7 fort bewegt werden kann.

Um die fluiddichte Anlage des Adapters 6 an den Schlauchanschlussstutzen 7 noch weiter zu verbessern, sind an den freien Enden der Längsschenkel 6a des H-förmigen Adapters 6 zum Abdichten gegenüber den Schlauchanschlussstutzen 7 Dichtungselemente 14, insbesondere in der Form von O-Ring-Dichtungen, festgelegt, die beim Anpressen des Adapters 6 gegen die Schlauchanschlussstutzen 7 abdichtend verformt werden.

Ebenso ist es möglich, die Dichtungselemente 14 auf Seiten der Schlauchanschlussstutzen 7 anzuordnen, um eine fluiddichte Anlage des Adapters 6 an den Schlauchanschlussstutzen 7 zu gewährleisten.

Alternativ zu der dargestellten Ausführungsform ist es selbstverständlich auch möglich, den Anschlag 13 am Basisteil 4 so anzuordnen, dass dieser beim Schlieβen des Deckels 2 gegen den Adapter 6 anläuft und den Adapter 6 abdichtend gegen die Schlauchanschlussstutzen 7 presst.

Ein solchermaßen ausgebildeter Handgriff zeichnet sich dadurch aus, dass er bei einfachem Aufbau leicht zu montieren und zu reinigen ist und bei jedem Schließen des Deckels 2 des Griffgehäuses 3 automatisch sicherstellt, dass die Schläuche 5 über den Adapter 6 fluiddicht an den Schlauchanschlussstutzen 7 angeschlossen sind.

### Bezugszeichenliste

- 1: Handgriff
- 2: Deckel
- 3: Griffgehäuse
- 4: Basisteil
- 5: Saug- und Spülschlauch / Schlauch
- 6: Adapter
- 6a: Längsschenkel
- 6b: Verbindungssteg
- 7: Schlauchanschlussstutzen
- 8: Taster
- 9: Kupplungselement
- 10: Instrumentenschaft
- 11: Sperrmechanismus
- 12: Schwenkachse
- 13: Anschlag
- 14: Dichtungselement

## Patentansprüche

1. Handgriff (1) für ein medizinisches Instrument welcher Handgiff mit einem über einen Deckel (2) verschließbaren Griffgehäuse (3) und mindestens einem im Griffgehäuse (3) an einen Schlauchanschlussstutzen (7) anschließbaren Saug- und/oder Spülschlauch (5) versehen ist, weiter aufweisend einen Adapter (6), wobei jeder Saug- und/oder Spülschlauch (5) über den Adapter (6) an den zugehörigen Schlauchanschlussstutzen (7) anschließbar ist,
**dadurch gekennzeichnet,**
**dass** der zum Anschließen von zwei Schläuchen (5) an zwei Schlauchanschlussstutzen (7) ausgebildete Adapter (6) beim Schließen des Griffgehäuses (3) über einen am Deckel (2) des Griffgehäuses (3) angeordneten und direkt gegen den Adapter (6) anlaufenden Anschlag (13) gegen die Schlauchanschlussstutzen (7) anpressbar ist, wobei der Adapter (6) H-förmig oder U-förmig ausgebildet ist.

2. Handgriff nach Anspruch 1, **dadurch gekennzeichnet, dass** der Anschlag (13) als am Deckel (2) angeformter, nach innen weisender Steg ausgebildet ist.

3. Handgriff nach Anspruch 1 oder 2, **dadurch gekennzeichnet, dass** zum Abdichten gegenüber dem Schlauchanschlussstutzen (7) am Adapter (6) mindestens ein Dichtungselement (14), insbesondere eine O-Ring-Dichtung, festlegbar ist.

4. Handgriff nach Anspruch 1 oder 2, **dadurch gekennzeichnet, dass** zum Abdichten gegenüber dem Adapter (6) am Schlauchanschlussstutzen (7) mindestens ein Dichtungselement (14), insbesondere eine O-Ring-Dichtung, festlegbar ist.

5. Handgriff nach Anspruch 1 oder 2, **dadurch gekennzeichnet, dass** zum Abdichten gegenüber dem Schlauchanschlussstutzen (7) der Adapter (6) aus einem Dichtungsmaterial, insbesondere Silikon, besteht.

## Claims

1. Handle (1) for a medical instrument, and which handle (1) comprises a handle housing (3) that can be closed via a lid (2) and at least one suction and/or rinse hose (5) that can be connected inside the handle housing (3) to a hose connecting branch and further comprising an adapter (6), and wherein each suction and/or rinse hose (5) can be connected to the associated hose connecting branch (7) via the adapter (6)
**characterized in that**
the adapter (6) that is configured to connect two hoses (5) to two hose connecting branches (7) can, during the closing action of the handle housing (3), be pressed against the hose connecting branch (7) via a stop (13) that is disposed on the lid (2) of the handle housing (3) running directly against the adapter (6), and wherein the adapter (6) is configured in an H-shape or U-shape.

2. Handle according to Claim 1 **characterized in that** the stop (13) is configured as a bar that is formed in one piece with the lid (2) and points toward the inside.

3. Handle according to Claim 1 or 2 **characterized in that** at least one sealing element can be provided on the adapter (6) for providing sealing action relative to the hose connecting branch (7), in particular an O-ring gasket.

4. Handle according to Claim 1 or 2 **characterized in that** at least one sealing element (14) can be localized on the hose connecting branch (7) for providing sealing action relative to the adapter (6), in particular an O-ring gasket.

5. Handle according to Claim 1 or 2 **characterized in that** the adapter (6) is comprised of a sealing material, in particular silicone, in order to provide sealing action relative to the hose connecting branch (7).

## Revendications

1. Poignée (1) pour un instrument médical dont la poignée est pourvue d'un boîtier de préhension (3) refermable par un couvercle (2) et d'au moins un tuyau d'aspiration et/ou de rinçage (5) raccordable à une tubulure de raccord de tuyau (7) dans le boîtier de préhension (3), comprenant en outre un adaptateur (6), chaque tuyau d'aspiration et/ou de rinçage (5) étant raccordable par ledit adaptateur (6) à la tubulure de raccord de tuyau (7) correspondante,
**caractérisée**
**en ce que** l'adaptateur (6) prévu pour le raccordement de deux tuyaux (5) à deux tubulures de raccord de tuyau (7) est serrable contre les tubulures de raccord de tuyau (7) à la fermeture du boîtier de préhension (3), au moyen d'une butée (13) disposée sur le couvercle (2) du boîtier de préhension (3) et butant directement contre l'adaptateur (6), ledit adaptateur (6) étant en forme de H ou de U.

2. Poignée selon la revendication 1, **caractérisée en ce que** la butée (13) est réalisée comme barrette formée sur le couvercle (2) et dirigée vers l'intérieur.

3. Poignée selon la revendication 1 ou la revendication 2, **caractérisée en ce qu'**au moins un élément d'étanchéité (14), en particulier un joint torique, est fixable sur l'adaptateur (6) pour réaliser une étanchéité par rapport à la tubulure de raccord de tuyau (7).

4. Poignée selon la revendication 1 ou la revendication 2, **caractérisée en ce qu'**au moins un élément d'étanchéité (14), en particulier un joint torique, est fixable sur la tubulure de raccord de tuyau (7) pour réaliser une étanchéité par rapport à l'adaptateur (6).

5. Poignée selon la revendication 1 ou la revendication 2, **caractérisée en ce que**, pour réaliser une étanchéité par rapport à la tubulure de raccord de tuyau (7), l'adaptateur (6) est composé d'un matériau de joint, en particulier de silicone.
